(19) Europäisches Patentamt
European Patent Office
Office européen des brevets

(11) Numéro de publication: **0 007 828**
**B1**

(12) **FASCICULE DE BREVET EUROPEEN**

(45) Date de publication du fascicule de brevet: **04.11.81**

(21) Numéro de dépôt: **79400443.2**

(22) Date de dépôt: **29.06.79**

(51) Int. Cl.[3]: **C 07 C 69/747,**
C 07 C 67/303
//C07D231/14, C07D231/06,
C07C69/52, C07C69/587,
C07C67/30

(54) **Procédé de préparation d'esters d'alcoyle de l'acide dl-cis chrysanthémique.**

(30) Priorité: **24.07.78 FR 7821814**

(43) Date de publication de la demande:
**06.02.80 Bulletin 80/3**

(45) Mention de la délivrance du brevet:
**04.11.81 Bulletin 81/44**

(84) Etats Contractants Désignés:
**CH DE FR GB NL**

(56) Documents cités:
**Néant**

(73) Titulaire: **ROUSSEL-UCLAF**
**102, route de Noisy Boîte postale no.9**
**F-93230 Romainville (FR)**

(72) Inventeur: **Franck-Neumann, Michel**
**4, rue Andrieux**
**F-67000 Strasbourg (FR)**
Inventeur: **Dietrich-Buchecker, Christiane**
**32, rue des Juifs**
**F-67000 Strasbourg (FR)**
Inventeur: **Miesch, Michel**
**42a rue des Grains**
**F-68200 Mulhouse (FR)**

(74) Mandataire: **Petranker, Léon**
**boîte postale no 9 102, route de Noisy**
**F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 007 828 B1

Procédé de préparation d'esters d'alcoyle de l'acide dl-cis chrysanthémique

La présente invention concerne un procédé de préparation d'esters d'alcoyle de l'acide dl-cis chrysanthémique.

L'invention a plus particulièrement pour objet, un procédé de préparation d'esters d'alcoyle de l'acide 2,2-diméthyl 3 RS-(2-méthyl 1-propényl) cyclopropane-1 SR-carboxylique ou esters d'alcoyle de l'acide dl cis chrysanthémique, esters de formule (I):

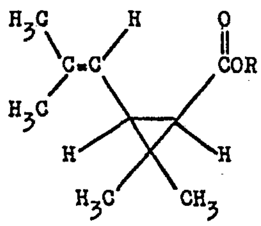

(I)

dans laquelle R représente un radical alcoyle comportant de 1 à 6 atomes de carbone, caractérisé en ce que l'on fait réagir un 3,3-diméthyl 2-(2-méthyl 1-propényl 1-alcoxy carbonyl cyclopropène de formule (VI):

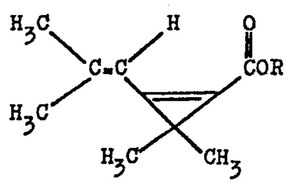

VI

dans laquelle R conserve la signification précitée avec un agent réducteur, choisi dans le groupe constitué par l'hydrogène en présence de borure de nickel ou de palladium déposé sur carbonate de calcium et le diimide.

Lorsqu'on effectue la réduction par l'hydrogène en présence d'un catalyseur, on utilise de préférence comme catalyseur le borure de nickel "P—1" préparé, in situ, selon la méthode de C. A. BROWN J. Org. Chem. *35,* 1900 (1970).

Lorsqu'on effectue la réduction par le diimide, on prépare, de préférence, ce réactif selon la méthode de E. E. VAN TAMELEN et col. J. Am. Chem. Soc. *83*, 3725 (1961).

Selon l'invention, le 3,3-diméthyl 2-(2-méthyl 1-propényl) 1-alcoxy carbonyl cyclopropène (VI) est obtenu en faisant réagir le méthyl lithium avec le 2-méthyl pent-2-èn 4-yne (II):

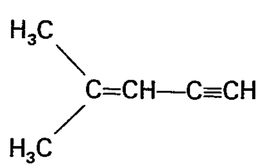

(II)

puis en soumettant le dérivé lithien résultant, à l'action d'un chloroformiate d'alcoyle, pour obtenir un ester d'alcoyle de l'acide 5-méthyl hex-4-èn 2-yne oïque (III) de formule:

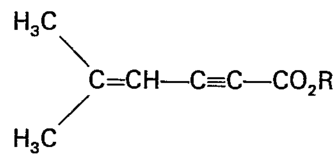

(III)

dans laquelle R représente un radical alcoyle comportant de 1 à 6 atomes de carbone, puis en faisant réagir ce composé avec le diazo-2-propane, pour obtenir un mélange d'ester d'alcoyle de l'acide 3,3-diméthyl 5-(2-méthyl 1-propényl) 3H-pyrazole 4-carboxylique (IV):

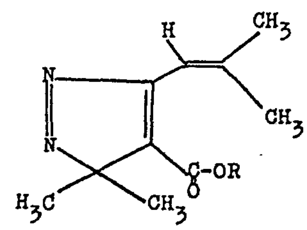

(IV)

et d'ester d'alcoyle de l'acide 3,3-diméthyl 4-(2-méthyl 1-propényl) 3H-pyrazole 5-carboxylique (V):

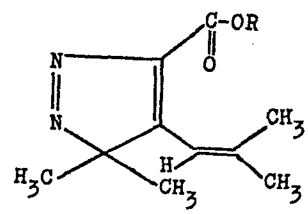

(V)

que l'on sépare, le cas échéant, puis en soumettant le composé de formule IV, le composé de formule V ou leur mélange à une irradiation, puis en isolant le composé VI attendu.

A l'issu du procédé ci-dessus, le produit attendu de formule VI peut être obtenu en mélange avec un produit de formule VII:

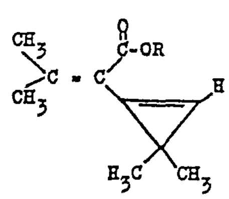

(VII)

De ce mélange de produits de formules VI et VII, on peut, le cas échéant, isoler le produit de

formule VI par des moyens usuels, tels que la chromatographie.

L'ester méthylique de l'acide 5-méthyl hex-4-èn 2-yne oïque est obtenu, de préférence, en introduisant une solution éthérée de dérivé lithien du 2-méthyl pent-2-èn 4-yne à −55°C ± 20°C, dans une solution éthérée de chloroformiate de méthyle.

Les autres esters d'alcoyle peuvent être préparés d'une manière analogue en utilisant un chloroformiate d'alcoyle convenable.

Le mélange d'ester méthylique de l'acide 3,3-diméthyl 5-(2-méthyl 1-propényl) 3H-pyrazole 4-carboxylique et d'ester méthylique de l'acide 3,3-diméthyl 4-(2-méthyl 1-propényl) 3H-pyrazole 5-carboxylique est obtenu, de préférence, en introduisant à −60°C ± 20°C, une solution éthérée de diazo-2-propane dans une solution éthérée d'ester méthylique de l'acide 5-méthyl hex-4-èn 2-yne oïque.

A partir des autres esters d'alcoyle de l'acide 5-méthyl hex-4-èn 2-yne oïque, on obtient de manière analogue des mélanges d'ester d'alcoyle de l'acide 3,3-diméthyl 5-(2-méthyl 1-propényl) 3H-pyrazole 4-carboxylique et d'ester d'alcoyle de l'acide 3,3-diméthyl 4-(2-méthyl 1-propényl) 3H-pyrazole 5-carboxylique.

Le 3,3-diméthyl 2-(2-méthyl 1-propényl) 1-(méthoxy carbonyl) cyclopropène (ou son mélange avec le 2-(3,3-diméthyl cyclopropan-1-en 2-yl) 3-métnyl 2-butanoate de méthyle) est obtenu par irradiation, de préférence, en utilisant une lampe à vapeur de mercure à moyenne ou haute pression dont la longueur d'onde du rayonnement est supérieure à 300 nm, telle qu'une lampe PHILIPS HPK 125, en opérant à + 15°C ± 10°C.

Le 2-méthyl pent-2-èn 4-yne utilisé au départ du procédé de l'invention, peut être préparé selon la méthode de A. MONDON (Ann. *577* 181—201, (1952) par chauffage du 2-méthyl 2-hydroxy pent-4-yne en présence de sulfate acide de potassium.

L'invention a également pour objet une variante du procédé de préparation des esters de formule I, caractérisée en ce que l'on soumet un composé de formule (III):

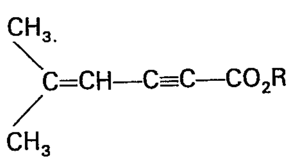

(III)

dans laquelle R est défini comme précédemment, à l'action d'un agent réducteur, pour obtenir un composé de formule (VIII):

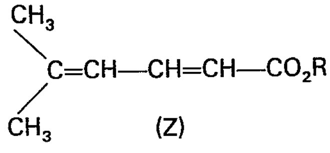

(VIII)

(Z)

que l'on fait agir avec le diazo-2-propane, pour obtenir un mélange d'ester d'alcoyle de l'acide cis 4,5-dihydro 3,3-diméthyl 5-(2-méthyl 1-propényl) 3H-pyrazole 4-carboxylique (IX):

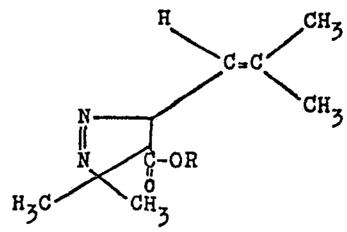

(IX)

et d'ester d'alcoyle de l'acide 4,5-dihydro 5,5-diméthyl 4-(2-méthyl 1-propényl) 1H-pyrazole 3-carboxylique (X):

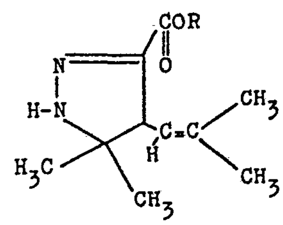

(X)

que l'on sépare, puis soumet le composé de formule (IX) à une irradiation ou à l'action de la chaleur, pour obtenir le composé de formule (I):

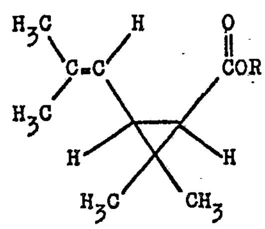

(I)

L'agent réducteur que l'on fait agir sur le composé (III) est de préférence l'hydrogène en présence du catalyseur de Lindlar (Palladium à 5% sur $CaCO_3$ additionné d'acétate de plomb), mais il peut être également l'un des autres agents déjà cités pour la réduction du composé (VI).

L'action du diazo-2-propane s'effectue dans les conditions préférentielles citées précédemment.

La séparation des composés de formules IX et X peut être effectuée par des moyens usuels tels que la chromatographie.

L'irradiation du composé (IX) est effectuée, de préférence, dans les conditions déjà décrites pour l'irradiation des composés IV et V.

Le chauffage du composé (IX) est effectué, de préférence, aux environs de 110°C, notamment au sein d'un solvant organique tel que le toluène.

Pendant de nombreuses années les spécialistes du domaine des insecticides s'intéressaient, surtout, à l'acide trans chrysanthémique car seuls les acides de configuration

trans étaient réputés conduire à des esters insecticides suffisamment actifs.

De nombreuses synthèses totales d'acides chrysanthémiques avaient été mises au point. Ces synthèses conduisaient, soit à des mélanges d'acides de structure dl cis et dl trans, pauvres en acides cis, soit à des acides de structure trans.

Les acides cis chrysanthémiques ont vu, depuis plusieurs années, leur importance croître. Ils sont utilisés, en effet, notamment pour préparer des acides cyclopropane carboxyliques à chaîne dihalovinylique dont les esters possèdent une activité insecticide bien supérieure à celle des esters d'acides trans-chrysanthémiques (voir par exemple BF 2.185.612 et 2.240.914).

La synthèse des acides cis chrysanthémiques présente donc, à l'heure actuelle, un intérêt tout particulier.

Le procédé de la présente invention fournit, pour la première fois, une synthèse totale stéréospécifique d'esters d'alcoyle d'acide cis-chrysanthémique racémique. Cette synthèse ne comporte qu'un nombre de stades restreint et son rendement global est élevé (de l'ordre de 60% ou plus).

Le procédé de l'invention présente des étapes originales sur le plan chimique.

Tout d'abord la réduction spécifique dans des conditions appropriées, de la double liaison endocyclique du dérivé cyclopropénique (VI), sans attaque de la double liaison de la chaîne latérale, conduisant d'une façon stéréospécifique, avec un rendement presque quantitatif, à l'ester de l'acide dl cis chrysanthémique désiré, constitue un type de réaction qui, à la connaissance de la société demanderesse, n'avait jamais été décrit à ce jour. Sans la réussite inattendue de cette réduction spécifique, tout le succès de la synthèse aurait été remis en cause.

De même, la réduction du composé III en composé VIII, dans les conditions décrites dans l'invention, est également très selective et permet ainsi l'accès à la structure pyrazolique dans d'excellentes conditions.

D'autre part, la fait que l'irradiation des deux dérivés pyrazoliques substitués (IV) et (V), ne conduise pratiquement qu'à un seul dérivé cyclopropénique, présente, également, un caractère original. C. DIETRICH-BUCHECKER et M. FRANCK NEUMANN (TETRAHEDRON vol. 33 p. 753—755 (1977)), avaient, il est vrai, décrit un phénomène analogue mais concernant des dérivés pyrazoliques dont les substituants étaient différents de ceux des composés (IV) et (V) de la présente demande. Rien ne permettait d'affirmer, à priori, que le phénomène constaté par ces auteurs fût transposable au cas où les substituants du pyrazole, comme dans la présente demande, sont un groupement alcoxy carbonyle et un groupement (2-méthyl 1-propényl).

Les exemples suivants illustrent l'invention, sans la limiter.

Exemple 1

Ester méthylique de l'acide 2,2-diméthyl 3 RS (2-méthyl 1-propényl) cyclopropan-1 SR-carboxylique ou ester méthylique de l'acide dl cis chrysanthémique.

*STADE A: Ester méthylique de l'acide 5-méthyl hex-4-ène 2-yne oïque.*

On prépare d'abord le dérivé lithien du 2-méthyl pent-2-ène 4-yne, en introduisant, progressivement, à —15°C, 40 ml de solution éthérée à 5% de méthyl lithium, sous atmosphère d'azote, dans un récipient refroidi à —15°C, puis en ajoutant 5 g de 4-méthyl 3-ène pent-1-yne, progressivement, à une température inférieure à 0°C.

On introduit ensuite, lentement, la solution de lithien précédemment obtenue, dans une solution de 7,5 cm3 de chloroformiate de méthyle dans 50 ml d'éther, en maintenant la température inférieure à —50°C, laisse remonter la température à +20°C, verse le mélange réactionnel sur de la glace, sépare par décantation la phase organique, extrait la phase aqueuse à l'éther, sèche la solution éthérée, la concentre à sec et obtient 7,86 g d'ester méthylique brut qui, par distillation sous vide de 0,1 mm de mercure, conduit à l'obtention de 7,44 g d'ester méthylique de l'acide 5-méthyl hex-4-ène 2-yne oïque.

*Stade B: Mélange d'ester méthylique de l'acide 3,3-diméthyl 5-(2-méthyl 1-propényl) 3H-pyrazole 4-carboxylique et d'ester méthylique de l'acide 3,3-diméthyl 4-(2-méthyl 1-propényl) 3H-pyrazole 5-carboxylique.*

Isolement des constituants du mélange.

On dissout 1 g d'ester méthylique de l'acide 5-méthyl hex-4-ène 2-yne oïque, obtenu au stade A, dans 20 cm3 d'éther, refroidit la solution à —60°C, ajoute progressivement 4 ml de solution de diazo-2-propane dans l'éthyl benzène à 2,14 m-moles/ml, obtenue selon une méthode analogue à celle décrite par C. DIETRICH-BUCHECKER et M. FRANCK-NEUMANN, Tétrahédron Vol 33, p.745 à 749 (1977), laisse la température remonter à 0°C, observe la disparition de la couleur rouge caractéristique du diazo-2-propane, ainsi qu'une montée de température vers +30°C, ajoute 1 ml de solution de diazo-2-propane, laisse la solution réactionnelle au repos pendant 4 heures, vers +20°C, concentre à sec par distillation sous pression réduite et obtient 1,70 g de mélange d'ester méthylique de l'acide 3,3-diméthyl 5-(2-méthyl 1-propényl) 3H-pyrazole 4-carboxylique et d'ester méthylique de l'acide 3,3-diméthyl 4-(2-méthyl 1-propényl) 3H-pyrazole 5-carboxylique (dans la proportion 2/1, d'après le spectre de RMN).

Par chromatographie sur gel de silice, en éluant avec un mélange d'éther de pétrole (éb. 40—60°C) et d'éther éthylique (9/1), on obtient 0,84 g d'ester méthylique de l'acide 3,3-diméthyl 5-(2-méthyl 1-propényl) 3H-pyrazole 4-carboxylique, puis en éluant avec un mélange d'éther de pétrole (éb. 40—60°C) et d'éther éthylique (6/4), on recueille 0,39 g d'ester méthylique de l'acide 3,3-diméthyl 4-(2-méthyl 1-propényl) 3H-pyrazole 5-carboxylique.

*Stade C: 3,3-diméthyl 2-(2-méthyl 1-propényl) 1-méthoxy carbonyl cyclopropène*

a) Irradiation de l'ester méthylique de l'acide 3,3-diméthyl 4-(2-méthyl 1-propényl) 3H-pyrazole 5-carboxylique.

L'irradiation est effectuée à +15°C, avec une lampe à vapeur de mercure PHILIPS HPK. 125, sous atmosphère d'azote.

On irradie 0,380 g d'ester méthylique de l'acide 3,3-diméthyl 4-(2-méthyl 1-propényl) 3H-pyrazole 5-carboxylique en solution dans 100 cm3 d'éther anhydre. Lorsque la quantité stoechiométrique d'azote s'est dégagée (≃45 ml), ce qui demande environ une heure, on arrête l'irradiation, évapore le solvant, obtient 0,350 g de produit brut (qui d'après le spectre de RMN contient uniquement le cyclopropène désiré), lequel, par chromatographie sur gel de silice, en éluant à l'éther de pétrole (éb. 40—60°C), fournit 0,315 g de 3,3-diméthyl 2-(2-méthyl 1-propényl) 1-(méthoxy carbonyl) cyclopropène.

b) Irradiation de l'ester méthylique de l'acide 3,3-diméthyl 5-(2-méthyl 1-propényl) 3H-pyrazole 4-carboxylique

L'irradiation de ce composé, dans les mêmes conditions que celles utilisées au paragraphe a) ci-dessus, conduit avec le même rendement au 3,3-diméthyl 2-(2-méthyl 1-propényl) 1-(méthoxy carbonyl) cyclopropène de même qualité que celui obtenu au paragraphe a) ci-dessus.

Le 3,3-diméthyl 2-(2-méthyl 1-propényl) 1-(méthoxy carbonyl) cyclopropène obtenu aux paragraphes a) ou b) ci-dessus présente les caractéristiques suivantes:

SPECTRE DE R.M.N.:

$\delta$ en p.p.m. par rapport au triméthyl silane, dans $CDCl_3$.

$CH_3$:

1,26 (6H singulet), 1,95 (6H doublet — Constante de couplage: J = 1,5 Hz)

$CH_3CO_2$:

3,75 (3H singulet), H vinylique: 6,28 (1H heptuplet, J ≃ 1,5 Hz).

c) L'irradiation du mélange d'ester méthylique de l'acide 3,3-diméthyl 5-(2-méthyl 1-propényl) 3H-pyrazole 4-carboxylique et d'ester méthylique de l'acide 3,3-diméthyl 4-(2-méthyl 1-propényl) 3H-pyrazole 5-carboxylique conduit, dans des conditions analogues à celles utilisées aux paragraphes a) et b) ci-dessus, à l'obtention du 3,3-dimethyl 2-(2-méthyl 1-propényl) 1-(méthoxy carbonyl) cyclopropène avec un rendement de l'ordre de 90%.

*STADE D: 2,2-diméthyl 3 RS-(2-méthyl 1-propényl) cyclopropane 1 SR-carboxylate de méthyle ou dl cis chrysanthémate de méthyle.*

a) Réduction par l'hydrogène en présence de borure de nickel "P—I"

Dans un appareil pour hydrogénation on prépare, in situ, selon la méthode de C.A. BROWN (J. Org. Chem *35* 1900 (1970)), le catalyseur au borure de nickel. On utilise 1,24 g de tétrahydrate d'acétate de nickel en solution dans l'éthanol, purge à plusieurs reprises, alternativement sous vide et sous hydrogène, agite sous atmosphère d'hydrogène, arrête l'agitation, introduit 1,0 g de 3,3-diméthyl 2-(2-méthyl 1-propényl) 1-(méthoxy carbonyl) cyclopropène, agite à nouveau sous atmosphère d'hydrogène, absorbe 125 cm3 d'hydrogène en trois heures environ, filtre le mélange réactionnel sur silicate de magnésium activé (florisil), pour éliminer le catalyseur, concentre le filtrat à sec et obtient 0,81 g de dl cis chrysanthémate de méthyle.

b) Réduction par le diimide

Le diimide est préparé, in situ, selon E. E. VAN TAMELEN et col. J. Amer. Chem. Soc. *83,* 3725 (1961) à partir de l'azodicarboxylate de potassium.

Dans une suspension de 1,4 g d'azodicarboxylate de potassium dans 80 cm3 de méthanol, on introduit, sous atmosphère d'azote, 1,15 g de 3,3-diméthyl 2-(2-méthyl 1-propényl) 1-méthoxy carbonyl cyclopropène, ajoute lentement 800 $\mu$l d'acide acétique, achève la dissolution du sel de potassium en ajoutant encore 100 $\mu$l d'acide acétique, concentre à sec la solution méthanolique, ajoute de l'eau au résidu, extrait la phase aqueuse au pentane, lave à l'eau les phases organiques réunies, les sèche, les concentre à sec et obtient 1,034 g d'ester dl cis brut. Par chromatographie sur gel de silice en éluant avec un mélange d'éther de pétrole (éb. 40—60°C) et d'éther éthylique (99/1) on obtient 0,838 g de dl cis chrysanthémate de méthyle.

Exemple 2

Ester méthylique de l'acide 2,2-diméthyl 3RS (2-méthyl 1-propényl) cyclopropane 1 SR-carboxylique ou ester méthylique de l'acide dl-cis chrysanthémique

*Stade A: Ester méthylique de l'acide 5-méthyl hex-2,4-diénoïque*

On hydrogène 500 mg de l'ester méthylique de l'acide 5-méthyl hex-4-ène 2-yne oïque (préparé à l'exemple 1) dans un appareil à hydrogéner, en présence de 150 mg de catalyseur de Lindlar, en suspension dans 100

cm3 d'acétate d'éthyle. Après 15 minutes d'hydrogénation, on obtient le produit attendu, filtre et utilise le mélange brut pour la suite de la synthèse.

*Stade B: Ester méthylique de l'acide 4,5-dihydro 3,3-diméthyl 5-(2-méthyl 1-propényl) 3H-pyrazole 4-carboxylique et ester méthylique de l'acide 4,5-dihydro 5,5-diméthyl 4-(2-méthyl 1-propényl) 1H-pyrazole 3-carboxylique*

L'addition de diazo-2-propane sur le produit obtenu précédemment s'effectue très lentement à 0°C. On ajoute un excès de diazo-2-propane dans le milieu réactionnel jusqu'à ce que la réaction soit complète. Le produit brut obtenu après évaporation du solvant est recristallisé à —78°C. dans un mélange éther/hexane. On isole 420 mg d'ester méthylique de l'acide 4,5-dihydro 5,5-diméthyl 4-(2-méthyl 1-propényl) 1H-pyrazole 3-carboxylique liquide à température ambiante. On chromatographie les liqueurs mères sur silice en éluant par un mélange éther/hexane (25.75) et isole 240 mg d'ester méthylique de l'acide 4,5-dihydro 3,3-diméthyl 5-(2-méthyl 1-propényl) 3H-pyrazole 4-carboxylique sous forme de liquide jaune pâle.

*Stade C: Ester méthylique de l'acide 2,2-diméthyl 3RS (2-méthyl 1-propényl) cyclopropane 1 SR-carboxylique ou ester méthylique de l'acide dl-cis chrysanthémique.*

— Après irradiation de 120 mg d'ester méthylique de l'acide 4,5-dihydro 3,3-diméthyl 5-(2-méthyl 1-propényl) 3H-pyrazole 4-carboxylique dans de l'éther anhydre, dans les conditions décrites à l'exemple 1, on obtient 100 mg de produit brut. On le chromatographie sur silice en éluant par un mélange éther/hexane (5:95) et recueille 95 mg de produit attendu, identique à celui obtenu à l'exemple 1.

— Par chauffage de l'ester méthylique de l'acide 4,5-dihydro 3,3-diméthyl 5-(2-méthyl 1-propényl) 3H-pyrazole 4-carboxylique à 110°C dans le toluène, on obtient également le produit attendu, renfermant cependant environ 30% de dérivé dl-trans.

**Revendications**

1. Procédé de préparation d'esters d'alcoyle de l'acide 2,2-diméthyl 3 RS-(2-méthyl 1-propényl) cyclopropane-1 SR-carboxylique ou esters d'alcoyle de l'acide dl cis chrysanthémique, esters de formule (I):

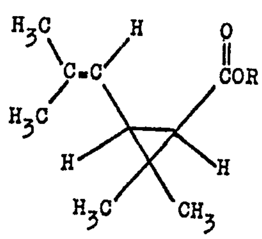

(I)

dans laquelle R représente un radical alcoyle comportant de 1 à 6 atomes de carbone, caractérisé en ce que l'on fait réagir un 3,3-diméthyl 2-(2-méthyl 1-propényl 1-alcoxy carbonyl cyclopropène de formula (VI):

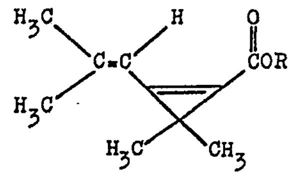

(VI)

dans laquelle R conserve la signification précitée, avec un agent réducteur choisi dans le groupe constitué par l'hydrogène en présence de borure de nickel ou de palladium déposé sur carbonate de calcium et le diimide.

2. Procédé de préparation selon la revendication 1, caractérisé en ce que l'hydrogénation est effectué en présence de borure de nickel "P—1", préparé, in situ, selon la méthode de C. A. BROWN J. Org. Chem. *35* 1900 (1970).

3. Procédé de préparation selon la revendication 1, caractérisé en ce que l'agent réducteur est le diimide, préparé selon la méthode de E. E. VAN TAMELEN et Col. J. Amer. Chem. Soc. *83*, 3725 (1961).

4. Procédé de préparation selon la revendication 1, caractérisé en ce que le 3,3-diméthyl 2-(2-méthyl 1-propényl) 1-alcoxy carbonyl cyclopropène (VI) est obtenu en faisant réagir le méthyl lithium avec le 2-méthyl pent-2-èn 4-yne (II):

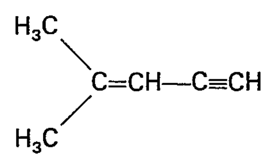

(II)

puis en soumettant le dérivé lithien résultant, à l'action d'un chloroformiate d'alcoyle, pour obtenir un ester d'alcoyle de l'acide 5-méthyl hex-4-èn 2-yne oïque (III) de formule:

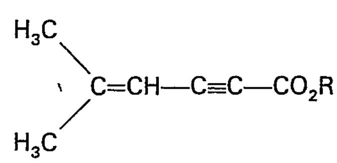

(III)

dans laquelle R est défini comme à la revendication 1, puis en faisant réagir ce composé avec le diazo-2-propane, pour obtenir un mélange d'ester d'alcoyle de l'acide 3,3-diméthyl 5-(2-méthyl 1-propényl) 3H-pyrazole 4-carboxylique (IV):

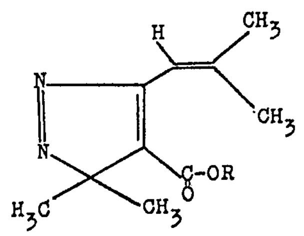

(IV)

et d'ester d'alcoyle de l'acide 3,3-diméthyl 4-(2-méthyl 1-propényl) 3H-pyrazole 5- carboxylique (V):

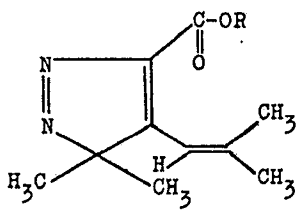

(V)

que l'on sépare, le cas échéant, puis en soumettant le composé de formule IV, le composé de formule V ou leur mélange à une irradiation, puis en isolant le composé VI attendu.

5. Procédé de préparation selon la revendication 4, caractérisé en ce que l'ester méthylique de l'acide 5-méthyl hex-4-èn 2-yne oïque est obtenu en introduisant une solution éthérée de dérivé lithien du 2-méthyl pent-2-èn 4-yne à — 55°C ± 20°C, dans une solution éthérée de chloroformiate de méthyle.

6. Procédé de préparation selon la revendication 4, caractérisé en ce que le mélange d'ester méthylique de l'acide 3,3-diméthyl 5-(2-méthyl 1-propényl) 3H-pyrazole 4-carboxylique et d'ester méthylique de l'acide 3,3-diméthyl 4-(2-méthyl 1-propényl) 3H-pyrazole 5-carboxylique est obtenu en introduisant à −60°C ± 20°C, une solution éthérée de diazo-2-propane dans une solution éthérée d'ester méthylique de l'acide 5-méthyl hex-4-èn 2-yne oïque.

7. Procédé de préparation selon la revendication 4, caractérisé en ce que le 3,3-diméthyl 2-(2-méthyl 1-propényl) 1-(méthoxy carbonyl) cyclopropène est obtenu par irradiation en utilisant une lampe à vapeur de mercure à moyenne ou haute pression dont la longueur d'onde du rayonnement est supérieure à 300 nm, telle qu'une lampe PHILIPS HPK 125, en opérant à +15°C ± 10°C.

8. Variante du procédé de préparation des esters de formule I, telle que définie à la revendication 1, caractérisée en ce que l'on soumet un composé de formule (III):

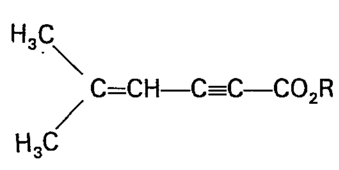

(III)

dans laquelle R est défini comme à la revendication 1, à l'action d'un agent réducteur, pour obtenir un composé de formule (VIII):

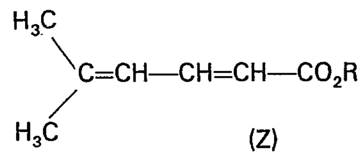

(Z)

(VIII)

que l'on fait agir avec le diazo-2-propane, pour obtenir un mélange d'ester d'alcoyle de l'acide cis 4,5-dihydro 3,3-diméthyl 5-(2-méthyl 1-propényl) 3H-pyrazole 4-carboxylique (IX):

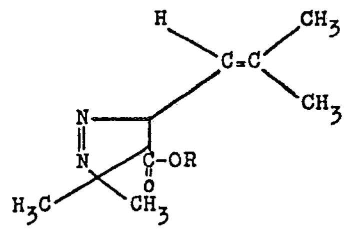

(IX)

et d'ester d'alcoyle de l'acide 4,5-dihydro 5,5-diméthyl 4-(2-méthyl 1-propényl) 1H-pyrazole 3-carboxylique (X):

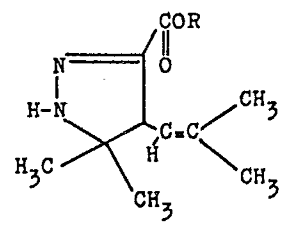

(X)

que l'on sépare, puis soumet le composé de formule (IX) à une irradiation ou à l'action de la chaleur, pour obtenir le composé de formule (I):

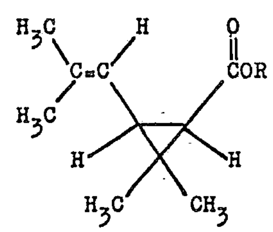

(I)

9. Procédé selon la revendication 8, caractérisé en ce que l'agent réducteur que l'on fait agir sur le composé (II) est l'hydrogène en présence du catalyseur de Lindlar.

10. Procédé selon la revendication 8, caractérisé en ce que l'irradiation est effectuée en utilisant une lampe à vapeur de mercure à moyenne ou haute pression dont la longueur donde du rayonnement est supérieure à 300 nm, telle qu'une lampe PHILIPS HPK 125, en opérant à +15°C ± 10°C.

11. Procédé selon la revendication 8,

caractérisé en ce que l'on obtient le composé de formùle (I) par action de la chaleur en chauffant aux environs de 110°C au sein d'un solvant organique.

## Claims

1. Process for preparing alkyl esters of 2,2-dimethyl 3 RS-(2-methyl 1-propenyl) cyclopropane-1 SR-carboxylic acid, or [in other words] alkyl esters of dl cis chrysanthemic acid, [these being] esters of formula (I):

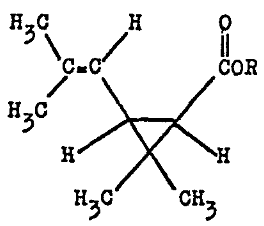
(I)

in which R represents an alkyl radical containing from 1 to 6 carbon atoms, characterised in that a 3,3-dimethyl 2-(2-methyl 1-propenyl) 1-alkoxy carbonyl cyclopropene of formula (VI):

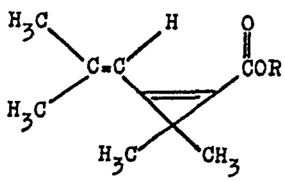
(VI)

in which R keeps the aforementioned meaning, is reacted with a reducing agent selected from the group constituted by nydrogen in the presence of nickel boride or of palladium deposited on calcium carbonate, and diimide.

2. Preparation process according to claim 1, characterised in that the hydrogenation is carried out in the presence of "P—1" nickel boride, prepared, *in situ*, according to the method of C.A. BROWN, J. Org. Chem., *35*, 1900 (1970).

3. Preparation process according to claim 1, characterised in that the reducing agent is diimide, prepared according to the method of E. E. VAN TAMELEN *et al.,* J. Amer. Chem. Soc. *83*, 3725 (1961).

4. Preparation process according to claim 1, characterised in that the 3,3-dimethyl 2-(2-methyl 1-propenyl) 1-alkoxy carbonyl cyclopropene (VI) is obtained by reacting methyl lithium with 2-methyl pent-2-en 4-yne (II):

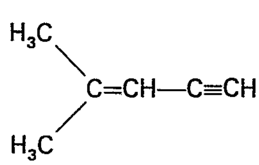
(II)

then by subjecting the resultant lithium derivative to the action of an alkyl chloroformate to obtain an alkyl ester of 5-methyl hex-4-en-2-ynoic acid (III) of formula:

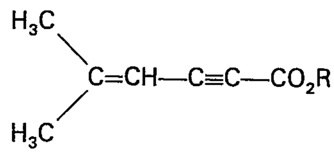
(III)

in which R is defined as in claim 1, then by reacting this compound with diazo-2-propane to obtain a mixture of the alkyl ester of 3,3-dimethyl 5-(2-methyl 1-propenyl) 3H-pyrazole 4-carboxylic acid (IV):

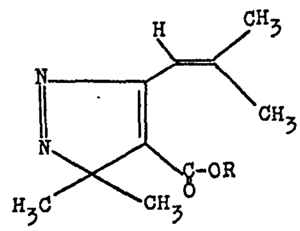
(IV)

and of the alkyl ester of 3,3-dimethyl 4-(2-methyl 1-propenyl) 3H-pyrazole 5-carboxylic acid (V):

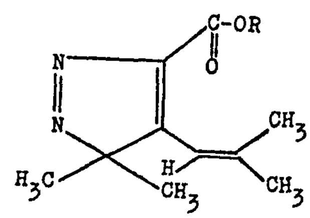
(V)

which is separated, if appropriate, then by subjecting the compound of formula IV, the compound of formula V or the mixture thereof to irradiation, then by isolating the expected compound VI.

5. Preparation process according to claim 4, characterised in that the methyl ester of 5-methyl hex-4-en-2-ynoic acid is obtained by introducing an ethereal solution of the lithium derivative of 2-methyl pent-2-en 4-yne into an ethereal solution of methyl chloroformate at $-55°C \pm 20°C$.

6. Preparation process according to Claim 4, characterised in that the mixture of the methyl ester of 3,3-dimethyl 5-(2-methyl 1-propenyl) 3H-pyrazole 4-carboxylic acid and of the methyl ester of 3,3-dimethyl 4-(2-methyl 1-propenyl) 3H-pyrazole 5-carboxylic acid is obtained by introducing an ethereal solution of diazo-2-propane into an ethereal solution of the methyl ester of 5-methyl hex-4-en-2-ynoic acid at $-60°C \pm 20°C$.

7. Preparation process according to Claim 4, characterised in that the 3,3-dimethyl 2-(2-methyl 1-propenyl) 1-(methoxy carbonyl) cyclopropene is obtained by irradiation using a mercury vapour lamp with average or high pressure, of which the wave-length of the radiation is greater than 300 nm, such as a PHILIPS HPK lamp, operating at +15°C ± 10°C.

8. Modification of the process for preparing the esters of formula I, as defined in Claim 1, characterised in that a compound of formula (III):

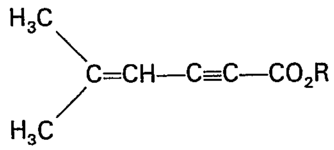
(III)

in which R is defined as in Claim 1, is subjected to the action of a reducing agent, to obtain a compound of formula (VIII):

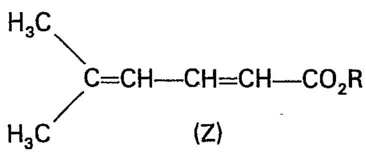
(VIII)
(Z)

which is reacted with diazo-2-propane, to obtain a mixture of the alkyl ester of cis 4,5-dihydro 3,3-dimethyl 5-(2-methyl 1-propenyl) 3H-pyrazole 4-carboxylic acid (IX):

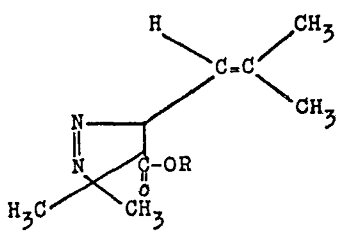
(IX)

and of the alkyl ester of 4,5-dihydro 5,5-dimethyl 4-(2-methyl) 1-propenyl) 1H-pyrazole 3-carboxylic acid (X):

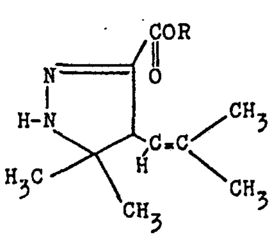
(X)

which is separated, then the compound of formula (IX) is subjected to irradiation or to the action of heat, to obtain the compound of formula (I):

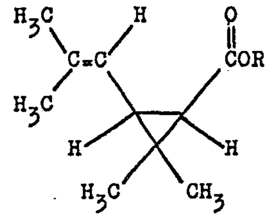
(I)

9. Process according to Claim 8, characterised in that the reducing agent reacted with the compound (II) is hydrogen in the presence of the Lindlar catalyst.

10. Process according to Claim 8, characterised in that the irradiation is carried out using a mercury vapour lamp with average or high pressure, of which the wave-length of the radiation is greater than 300 nm, such as a PHILIPS HPK 125 lamp, operating at +15°C ± 10°C.

11. Process according to Claim 8, characterised in that the compound of formula (I) is obtained by the action of heat by heating to about 110°C in an organic solvent.

**Patentansprüche**

1. Verfahren zur Herstellung von Alkylestern der 2,2 - Dimethyl - 3 - RS - (2 - methyl - 1 - propenyl) - cyclopropan - 1SR - carbonsäure oder von Alkylestern der D,L-cis-Chrysanthemumsäure, Estern der Formel I

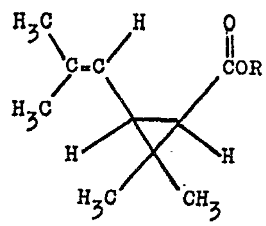
I

worin R einen Alkylrest mit 1 bis 6 Kohlenstoffatomen bedeutet, dadurch gekennzeichnet, daß man ein 3,3-Dimethyl - 2 - (2 - methyl - 1 - propenyl) - 1 - alkoxycarbonyl - cyclopropen der Formel VI

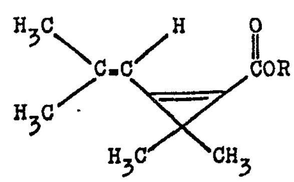
VI

worin R die vorstehend angegebene Bedeutung besitzt, mit einem Reduktionsmittel, ausgewählt aus der Gruppe, bestehend aus Wasserstoff in Gegenwart von Nickelborid oder aus auf Calciumcarbonat abgeschiedenem Palladium und Diimid, umsetz.

2. Herstellungsverfahren gemäß Anspruch 1, dadurch gekennzeichnet, daß die Hydrierung in

Gegenwart von Nickelborid "P—1", hergestellt in situ gemäß der Methode von C. A. BROWN J. Org. Chem, *35*, 1900 (1970) durchgeführt wird.

3. Herstellungsverfahren gemäß Anspruch 1, dadurch gekennzeichnet, daß das Reduktionsmittel Diimid, hergestellt nach der Methode von E. E. VAN TAMELEN et al J. Amer. Chem. Soc. *83*, 3725 (1961) ist.

4. Herstellungsverfahren gemäß Anspruch 1, dadurch gekennzeichnet, daß das 3,3 - Dimethyl - 2 - (2 - methyl - 1 - propenyl) - 1 - alkoxycarbonyl - cyclopropen (VI) erhalten wird, indem man Methyllithium mit 2-Methyl-pent-2-en-4-in (II)

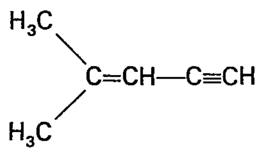

(II)

umsetzt, anschließend das erhaltene Lithiumderivat der Einwirkung eines Alkylchlorformiats unterzieht, um einen Alkylester der 5-Methyl-hex-4-en-2-in-säure (III) der Formel

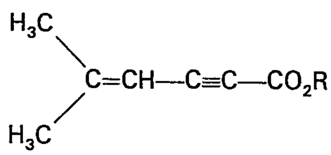

(III)

worin R wie Anspruch 1 definiert ist, zu erhalten, anschließend diese Verbindung mit Diazo-2-propan umsetzt, um ein Gemisch des Alkylesters der 3,3 - Dimethyl - 5 - (2 - methyl - 1 - propenyl - 3H - pyrazol - 4 - carbonsäure (IV)

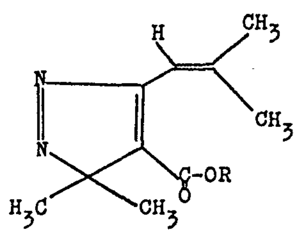

IV

und des Alkylesters der 3,3 - Dimethyl - 4 - (2 - methyl - 1 - propenyl) - 3H - pyrazol - 5 - carbonsäure (V)

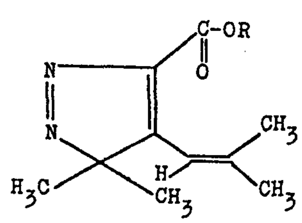

V

zu erhalten, das man gegebenenfalls trennt, anschließend die Verbindung der Formel IV, die Verbindung der Formel V oder deren Gemisch einer Bestrahlung unterzieht, wobei man danach die erwartete Verbindung VI isoliert.

5. Herstellungsverfahren gemäß Anspruch 4, dadurch gekennzeichnet, daß der Methylester der 5 - Methyl - hex - 4 - en - 2 - insäure erhalten wird, indem man eine ätherische Lösung des Lithiumderivats von 2 - Methyl - pent - 2 - en - 4 - in von $-55°C \pm 20°C$ in eine ätherische Methylchlorformiatlösung einbringt.

6. Herstellungsverfahren gemäß Anspruch 4, dadurch gekennzeichnet, daß das Gemisch des Methylesters der 3,3 - Dimethyl - 5 - (2 - methyl - 1 - propenyl) - 3H - pyrazol - 4 - carbonsäure und des Methylesters der 3,3 - Dimethyl - 4 - (2 - methyl - 1 - propenyl) - 3H - pyrazol - 5 - carbonsäure erhalten wird, indem man bei $-60°C \pm 20°C$ eine ätherische Diazo-2-propanlösung in eine ätherische Lösung des Methylesters der 5 - Methyl - hex - 4 - en - 2 - in - säure einbringt.

7. Herstellungsverfahren gemäß Anspruch 4, dadurch gekennzeichnet, daß das 3,3 - Dimethyl - 2 - (2 - methyl - 1 - propenyl) - 1 - (methoxycarbonyl) - cyclopropen durch Bestrahlung erhalten wird, indem man eine Quecksilberdampflampe von mittlerem oder hohem Druck, deren Strahlungswellenlänge oberhalb 300 nm liegt, wie eine PHILIPS HPK 125 Lampe verwendet, wobei man bei $+15°C \pm 10°C$ arbeitet.

8. Abänderung des Verfahrens zur Herstellung der Ester der Formel I gemäß Anspruch 1, dadurch gekennzeichnet, daß man eine Verbindung der Formel III

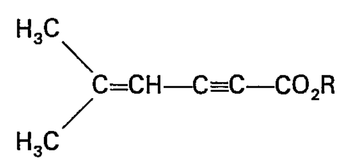

(III)

worin R wie in Anspruch 1 definiert ist, der Einwirkung eines Reduktionsmittels unterzieht, um eine Verbindung der Formel VIII

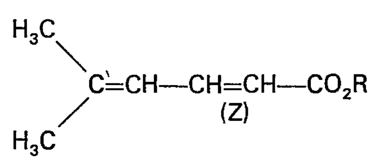

(VIII)

zu erhalten, die man mit Diazo-2-propan umsetzt, um ein Gemisch des Alkylesters der cis - 4,5 - Dihydro - 3,3 - dimethyl - 5 - (2 - methyl - 1 - propenyl) - 3H - pyrazol - 4 - carbonsäure (IX)

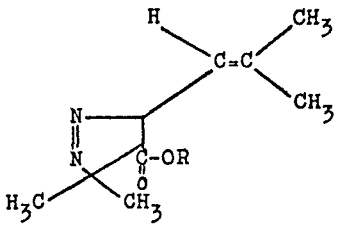

IX

und des Alkylesters der 4,5 - Dihydro - 5,5 - dimethyl - 4 - (2 - methyl - 1 - propenyl) - 1H - pyrazol - 3 - carbonsäure (X)

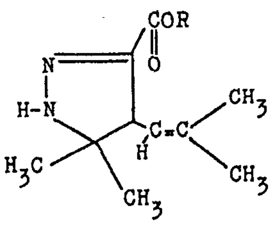

(X)

zu erhalten, das man auftrennt, anschließend die Verbindung der Formel IX einer Bestrahlung oder der Einwirkung von Wärme unterzieht, um die Verbindung der Formel I

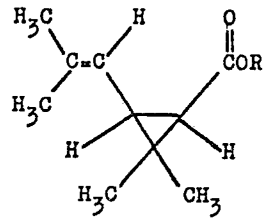

I

zu erhalten.

9. Verfahren gemäß Anspruch 8, dadurch gekennzeichnet, daß das Reduktionsmittel, das man mit der Verbindung (II) umsetzt, Wasserstoff in Gegenwart eines Lindlar-Katalysators ist.

10. Verfahren gemäß Anspruch 8, dadurch gekennzeichnet, daß die Bestrahlung durchgeführt wird, indem man eine Quecksilberdampflampe mit mittlerem oder hohem Druck, deren Strahlungswellenlänge oberhalb 300 nm liegt, wie eine PHILIPS HPK 125 Lampe verwendet, wobei man bei +15°C ± 10°C arbeitet.

11. Verfahren gemäß Anspruch 8, dadurch gekennzeichnet, daß man die Verbindung der Formel I durch Einwirkung von Wärme erhält, indem man auf etwa 110°C in dem Medium eines organischen Lösungsmittels erhitzt.